# EUROPEAN PATENT SPECIFICATION

(11) **EP 2 711 042 B1**
(45) Date of publication and mention of the grant of the patent: **26.06.2019**
(21) Application number: 12786221.7
(22) Date of filing: 10.05.2012
(51) Int. Cl.: B29D 23/00, A61B 8/12, A61B 8/00, A61M 25/00, A61M 25/01

(54) **MEDICAL TUBE, CATHETER, AND METHOD FOR PRODUCING MEDICAL TUBE**
MEDIZINISCHER SCHLAUCH, KATHETER UND VERFAHREN ZUR HERSTELLUNG DES MEDIZINISCHEN SCHLAUCHS
TUBE MÉDICAL, CATHÉTER ET PROCÉDÉ DE FABRICATION DE TUBES MÉDICAUX

(30) Priority: 17.05.2011 JP 2011110643
(43) Date of publication of application: 26.03.2014
(73) Proprietor: Terumo Kabushiki Kaisha, Tokyo 151-0072 (JP)
(72) Inventor: YAMASHITA, Yasunori, Fujinomiya-shi, Shizuoka 418-0015 (JP); OOTA, Tooru, Fujinomiya-shi, Shizuoka 418-0015 (JP); ITOU, Youichi, Fujinomiya-shi, Shizuoka 418-0015 (JP); KATOU, Naoko, Fujinomiya-shi, Shizuoka 418-0015 (JP)
(74) Representative: Dossmann, Gérard
(86) International application number: PCT/JP2012/061990
(87) International publication number: WO 2012/157513

(56) References cited:
- EP-A1- 1 955 724
- EP-A2- 0 958 911
- WO-A2-2008/006111
- JP-A- 2001 299 925
- JP-A- 2007 000 358
- JP-A- 2008 229 160
- JP-A- 2008 229 160

## Description

### Technical Field

The present invention relates to a medical tube, a catheter and a method of manufacturing a medical tube.

### Background Art

There is known a catheter which is used for diagnosing the inside of a body lumen by ultrasonic waves by inserting into the body lumen such as a blood vessel (for example, refer to Patent Literature 1).

The catheter described in Patent Literature 1 includes a catheter main body having a lumen, and a shaft which is inserted into the lumen of the catheter main body and has a ultrasonic oscillation unit which emits ultrasonic waves at the distal end portion. The catheter makes the shaft which is inserted into the lumen of the catheter main body, rotated around the axis thereof, as well as moved in the proximal end direction, and it is then possible to obtain an ultrasonic image of a blood vessel wall. In addition, when the ultrasonic image is obtained, the operation thereof is performed by filling the lumen, that is, in a gap which is formed between the inner circumferential surface of the catheter main body and the outer circumferential surface of the shaft with a liquid , for example, such as physiological salt solution. This gap is to some extent secured larger, to the extent that when filling with a liquid, the filling can be easily and quickly performed.

In a case where the catheter main body is configured by a resin, as a member of the proximal end side of the catheter main body, a tube in which the outer diameter is relatively large in order to secure sufficient rigidity is used. On the other hand, as a member of the distal end side of the catheter main body, a tube in which the outer diameter is relatively small is used, from the facility of inserting into the body lumen, or the like. Then, these tubes are jointed to form the catheter main body. Therefore, the outer diameter of the distal end portion of the tube of the proximal end side needs to be smaller in accordance with the size of the tube of the distal end side.

In addition, the inner diameter of each tube needs to be set to the predetermined value or more so that the resistance does not become big when filling in a gap between the inner circumferential surface of the catheter main body and the outer circumferential surface of the shaft with a liquid.

In addition, by the above-described tube of the proximal end side having the relatively large outer diameter at the proximal end side thereof and being configured such that the outer diameter of the distal end portion is relatively small, the reachability to the deep blood vessel and the flexibility are enhanced, and there is a need to improve priming performance by setting the inner diameter to the predetermined value or more.

As a method of manufacturing a medical tube like the above-described tube of the proximal end side, for example, a method of performing grinding, a method of performing thermoforming, and the like are included, with respect to a tube for processing having a certain outer diameter and a certain inner diameter.

However, in a method of grinding the outer circumferential surface of the tube for processing, since the outer diameter of the tube for processing does not become the inner diameter or less and the thickness becomes too thin, there is a risk of breaking the tube when using.

In addition, in a method of decreasing the outer diameter and the inner diameter at the same time, by performing thermoforming, with respect the tube for processing, it is difficult to make the outer diameter small while securing the inner diameter to the predetermined value or more.

PTL 1: Japanese Patent No. 3954888

JP 2008 229160 discloses a catheter capable of inhibiting extension of the distal end part and increasing the flow of a medical solution while maintaining the capability of reaching the periphery, or a catheter capable of inhibiting extension of the distal end and improving the capability of reaching the periphery while maintaining the flow of a medical solution.

One characteristic of the catheter is that a catheter tube with a distal end part and a proximal part has an axial direction member inside the catheter tube wall, and that the inner diameter of the distal end part is smaller than the inner diameter of the proximal part.

Another characteristic of the catheter is that the Shore hardness of the resin forming the catheter tube layer is gradually lowered from the proximal part to the distal end part of the catheter tube and that the length in the axial direction of layers in respective stages is varied.

### Disclosure of Invention

An object of the present invention is to provide a medical tube in which the outer diameter of the proximal end side is relatively large and the outer diameter of the distal end side is relatively small while securing the inner diameter having a sufficient size at the distal end side, a catheter, and a method of manufacturing a medical tube.

To realize the object described above, a medical tube according to the present invention is provided which includes a first site, a second site and a third site in this order from the proximal end side toward the distal end side and is made of resin,
in which at the second site, the outer diameter thereof is smaller than the outer diameter of the first site and the inner diameter is substantially equal to the inner diameter of the first site, and
in which at the third site, the outer diameter and the inner diameter thereof are respectively smaller than the outer diameter and the inner diameter of the second site, and wherein the first site, the second site, and the third site are integrally formed of the same material.

In a medical tube according to the present invention, it is preferable to have a first taper unit of which the outer diameter is gradually decreased from the proximal end side toward the distal end side between the first site and the second site.

In a medical tube according to the present invention, it is preferable to have a second taper unit of which the outer diameter and the inner diameter are, respectively, gradually decreased from the proximal end side toward the distal end side between the second site and the third site.

In a medical tube according to the present invention, it is preferable that an outer circumference of the second site and third site be a site in which other tube is coated.

In a medical tube according to the present invention, it is preferable that the thickness of the second site and the thickness of the third site be substantially equal.

In a medical tube according to the present invention, it is preferable that the thickness of the second site be 11% to 83% of the thickness of the first site.

In a medical tube according to the present invention, it is preferable that the outer diameter of the second site be 76% to 88% of the outer diameter of the first site.

In a medical tube according to the present invention, it is preferable that the outer diameter of the third site be 79% to 96% of the outer diameter of the second site.

In a medical tube according to the present invention, it is preferable that the inner diameter of the third site be 78% to 99% of the inner diameter of the second site.

In a medical tube according to the present invention, it is preferable that the outer diameter of the third site and the inner diameter of the second site be substantially equal.

In a medical tube according to the present invention, the first site, the second site, and the third site are integrally formed of the same material.

In a medical tube according to the present invention, it is preferable that the medical tube be configured by a thermoplastic resin and have flexibility.

In addition, to realize the object described above, a catheter according to the present invention is provided in which a catheter main body is insertable into a body lumen to be used and has flexibility, wherein the catheter main body has the medical tube according to the invention.

In a catheter according to the present invention, it is possible that a lumen of the medical tube can be used as a sensor lumen into which a driveshaft having an image fetching unit which is capable of fetching an image of the inside of the body lumen can be inserted at the distal end side.

In a catheter according to the present invention, it is preferable that the catheter main body be arranged in parallel with respect to the sensor lumen and have a guide wire lumen into which a guide wire is inserted.

In addition, to realize the object described above, a method of manufacturing the medical tube according to the present invention includes,
a first process of physically or chemically grinding the outer circumferential surface of the distal end portion of a tube for processing; and
a second process for conducting thermoforming, with respect to the site of the distal end side from a middle of a ground site of the tube for processing, and more reducing the outer diameter and the inner diameter of the site of the distal end side from the middle of the ground site than those of the site of the proximal end side from the middle of the ground site,
in which in the first process, the site which is not ground becomes the first site and the site which is ground becomes the second site, and
in which in the second process, the third site is formed by performing the thermoforming.

### Brief Description of Drawings

Fig. 1 is a partial longitudinal cross-sectional side view showing an embodiment of a catheter assembly including a catheter of the present invention.
Fig. 2 is an enlarged longitudinal cross-sectional view of an area [A] surrounded with a broken line in Fig. 1.
Fig. 3 is a longitudinal cross-sectional view showing a first tube in a catheter assembly shown in Fig. 1, that is, the first embodiment of a medical tube of the present invention.
Fig. 4 is a longitudinal cross-sectional view for explaining a manufacturing process of the first tube shown in Fig. 1.
Fig. 5 is an enlarged longitudinal cross-sectional view of an area [B] surrounded with a broken line in Fig. 2.

### Best Mode for Carrying Out the Invention

Hereinafter, detailed description will be typically given of a medical tube, a catheter and a method of manufacturing a medical tube of the present invention, based on preferred embodiments shown in attached drawings. Note that a use of a medical tube of the present invention is not particularly limited, however, in an embodiment described later, description will be given of a case where a medical tube of the present invention is applied to a constituent member of a catheter, that is, a first tube which configures a part of a catheter main body.

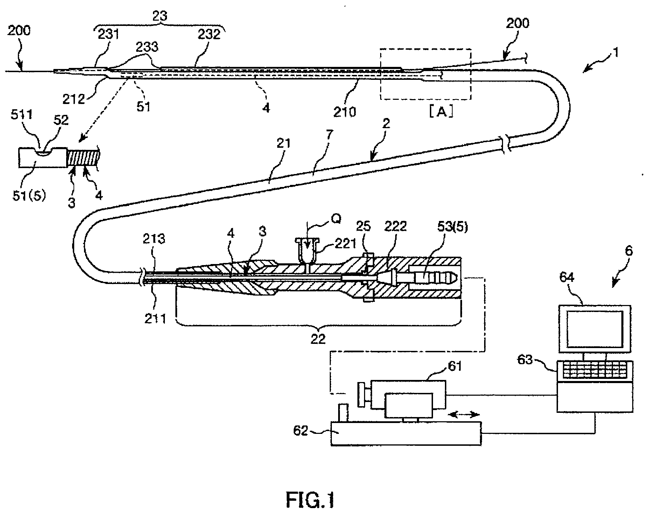

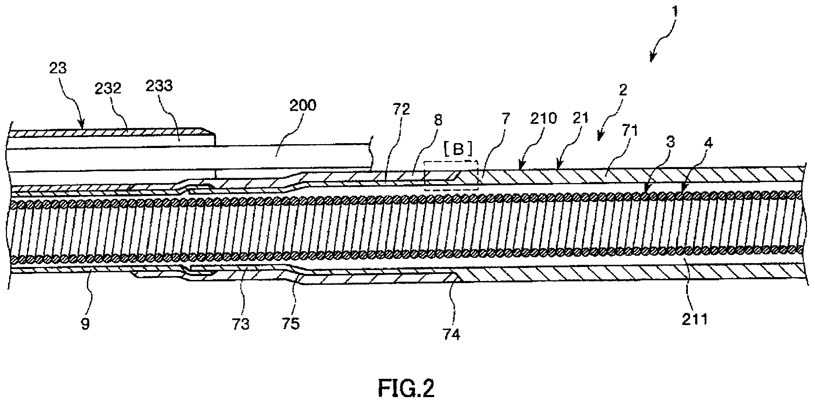

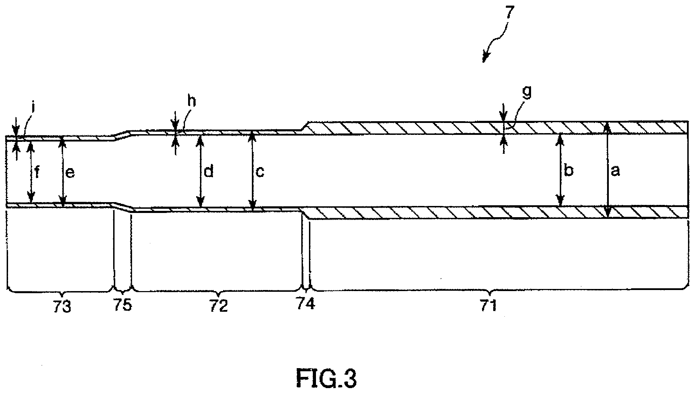

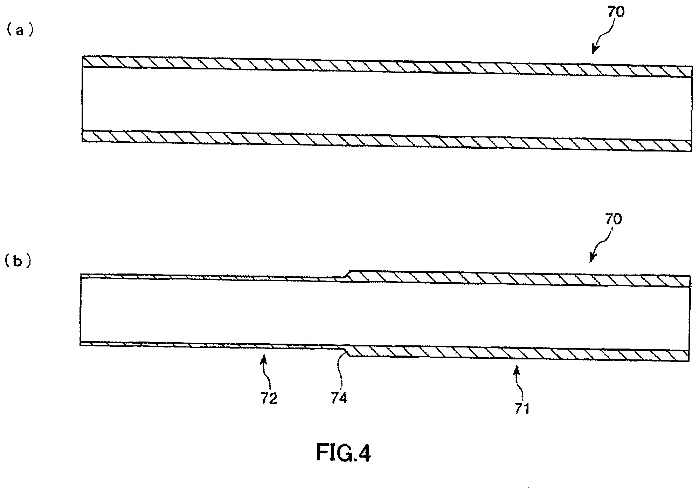

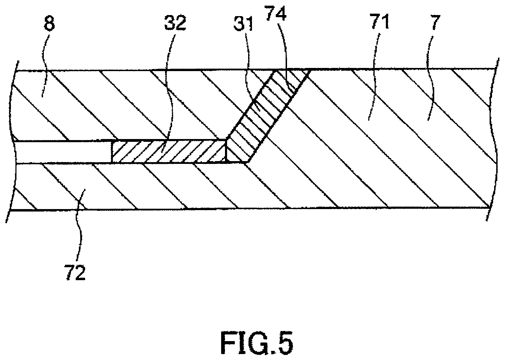

Fig. 1 is a partial longitudinal cross-sectional side view showing an embodiment of a catheter assembly including a catheter of the present invention, Fig. 2 is an enlarged longitudinal cross-sectional view of an area [A] surrounded with a broken line in Fig. 1, Fig. 3 is a longitudinal cross-sectional view showing a first tube in a catheter assembly shown in Fig. 1, that is, the first embodiment of a medical tube of the present invention, Fig. 4 is a longitudinal cross-sectional view for explaining a manufacturing process of the first tube shown in Fig. 1, and Fig. 5 is an enlarged longitudinal cross-sectional view of an area [B] surrounded with a broken line in Fig. 2.

Note that, hereinafter, description will be given of the left side, the right side, the upper side and the lower side in Fig. 1 to Fig. 5 as "the distal end", "the proximal end", "the upper" and "the lower", respectively.

A catheter assembly 1 shown in Fig. 1 and Fig. 2 includes a catheter 2 and a driveshaft 3 which is inserted into the catheter 2. The catheter assembly 1 is in an assembled state in which the catheter 2 and the driveshaft 3 are assembled, and is used by inserting into the body lumen (Hereinafter, typically dealt with "a blood vessel") to obtain an image of the blood vessel wall which is an internal image thereof.

In addition, this catheter assembly 1 is used by connecting an external unit 6. The external unit 6 consists of a scanner apparatus 61 which has an external driving source such as a motor built-in, an axial direction moving apparatus 62 which holds the scanner apparatus 61 to move in the horizontal direction (in the axial direction) by a motor, or the like, a control unit 63 which has a function of controlling a motion of the scanner apparatus 61 and the axial direction moving apparatus 62, and a display unit 64 which displays an image of the blood vessel wall obtained by the catheter assembly 1.

Description will be given of the external unit 6 before description will be given of a configuration of each part of the catheter assembly 1.

The scanner apparatus 61 is connected to the proximal end portion of the catheter assembly 1. Then, the scanner apparatus 61 can make the driveshaft 3 rotate around the axis thereof as well as is capable of moving with the catheter assembly 1 along the axial direction by the axial direction moving apparatus 62. In this manner, it is possible to scan an ultrasonic vibrator (an image fetching unit) 52 of the driveshaft 3. In addition, it is possible to form an image of the blood vessel wall in the external unit 6, based on information which is obtained from the driveshaft 3 which is sent through the scanner apparatus 61. In this manner, it is possible to obtain a transverse image in the blood vessel, which is an ultrasonic image, at an arbitrary position with respect to the blood vessel over whole circumference of the circumferential direction thereof.

The control unit 63, for example, is a personal computer which has a CPU (Central Processing Unit) built-in.

The display unit 64, for example, is a liquid crystal display apparatus.

Next, description will be given of the catheter assembly 1.

As described before, the catheter assembly 1 includes the catheter 2 and the driveshaft 3.

The catheter 2 includes a catheter main body 21 which has flexibility and has a long shape, and a connector unit 22 which is fixed to the proximal end portion of the catheter main body 21.

In the catheter main body 21, a sensor lumen 211 into which the driveshaft 3 is inserted and a guide wire lumen 233 into which a guide wire 200 is inserted are formed along the longitudinal direction of the catheter main body 21. In addition, the distal end portion of the catheter main body 21 becomes a reduced diameter unit in which the outer diameter is diametrically reduced.

The driveshaft 3 is capable of being inserted into the sensor lumen 211 and the sensor lumen 211 is formed throughout the full length of the catheter main body 21. Note that the site which is provided with the sensor lumen 211 in the catheter main body 21, it configures a driveshaft insertion unit 210 into which the driveshaft 3 is capable of being inserted.

The driveshaft insertion unit 210 has a first tube 7 which has flexibility, a second tube 8 which is provided at the proximal end side of the first tube 7 and has flexibility, and a third tube 9 which is provided at the proximal end side of the second tube 8 and has flexibility. Note that the outer diameter and the inner diameter of the third tube 9 are respectively smaller than the outer diameter and the inner diameter of a first site 71 described later of the first tube 7.

The first tube 7 is an embodiment of a medical tube according to the present invention, and configures from the proximal end until the middle of the distal end side in the driveshaft insertion unit 210. This first tube 7 has a first site 71, a second site 72 and a third site 73 in this order from the proximal end side toward the distal end side, as described later (refer to Fig. 3). In addition, the third tube 9 configures a part of the distal end site in the driveshaft insertion unit 210 and is fixed to the distal end portion of the first tube 7.

In this case, the inner circumferential surface of the proximal end portion of the third tube 9 is fixed to the outer circumferential surface of the distal end portion of the third site 73 of the first tube 7. Then, the second tube 8 covers the second site 72 and the third site 73 of the first tube 7 and the proximal end portion of the third tube 9. In this case, the inner circumferential surface of the proximal end side the of the second tube 8 is fixed to the outer circumferential surface of the second site 72 of the first tube 7, and the inner circumferential surface of the distal end side of the second tube 8 is fixed to the outer circumferential surface of the third site 73 of the first tube 7 and the outer circumferential surface of the proximal end portion of the third tube 9. Note that by the inner circumferential surface of the proximal end portion of the third tube 9 being fixed to the outer circumferential surface of the distal end portion of the third site 73 of the first tube 7, it is possible to prevent kinks in the catheter main body 21. A method of fixing of each unit described above is not particularly limited, and for example, adhesion by an adhesive agent, fusion such as heat fusion and ultrasonic fusion, and the like are included. In addition, the proximal end side end portion of the second tube 8 is butted to the distal end side end portion of the first site 71. Note that detailed description will be given of the first tube 7 later.

In addition, the guide wire 200 is capable of being inserted into the guide wire lumen 233, and this is formed only at the distal end portion of the catheter main body 21 in the present embodiment. Note that the site provided with the guide wire lumen 233 in the catheter main body 21 configures a guide wire insertion unit 23 into which the guide wire 200 is capable of being inserted.

A liquid Q is filled in the sensor lumen 211 in a state in which the driveshaft 3 is inserted, that is, in an insertion state. By the liquid Q being filled, ultrasonic waves from the ultrasonic vibrator 52 are transmitted to the blood vessel wall and it is possible to return back to the ultrasonic vibrator 52 from the blood vessel wall again. In this manner, it is possible to certainly obtain an ultrasonic image. Note that a liquid Q is not particularly limited, however, for example, physiological salt solution, a contrast medium, and the like are included.

In addition, the sensor lumen 211 has an opening unit 212 which is open at the distal end of the catheter main body 21. The liquid Q which is filled in the sensor lumen 211 is discharged through the opening unit 212. In this manner, even the liquid Q is excessively filled, it is possible to certainly prevent a break of the catheter main body 21 caused thereby.

Note that the opening unit 212 is open toward a sloping direction with respect to the central axis of the catheter main body 21 in a configuration shown in Fig. 1, however, is not limited thereto, and for example, may be open toward the central axis direction of the catheter main body 21, that is, the distal end direction.

The catheter main body 21 has the guide wire insertion unit 23 into which the guide wire 200 is capable of being inserted, at the distal end portion thereof. The guide wire insertion unit 23 is configured by two insertion members 231 and 232 which form a tubular-shape in which both ends are respectively open. Each insertion member 231 and 232 is arranged so as to separate with each other along a longitudinal direction of the catheter main body 21. Note that the guide wire lumen 233 is configured by the lumen of the guide wire insertion unit 23 and this guide wire lumen 233 is arranged in parallel with respect to the sensor lumen 211. The catheter 2 is inserted into the blood vessel in a state in which the guide wire 200 is inserted into the guide wire lumen 233 of the guide wire insertion unit 23 and is a catheter, so-called "rapid exchange type (short monorail type)" in which it is possible to rapidly perform the extraction and the insertion of the guide wire 200.

The guide wire insertion unit 23 is arranged parallel to the central axis of the catheter main body 21 in a configuration shown in Fig. 1, however, is not limited thereto, and for example, may be arranged to be tilted, respect to the central axis of the catheter main body 21.

In addition, in the middle in the longitudinal direction of the insertion member 231 of the distal end side of the guide wire insertion unit 23, a coil (not shown) is embedded. This coil functions as a contrast marker to visually recognize a position of the distal end portion of the catheter 2 under an X-ray illumination. Note that the coil is configured by a metallic material for example, like platinum having radiopaque properties.

The catheter main body 21 is configured by materials having flexibility, the materials thereof are not particularly limited, and for example, thermoplastic resins such as various kinds of thermoplastic elastomers such as styrene-based, polyolefin-based, polyurethane-based, polyester-based, polyamide-based, polyimide-based, polybutadiene-based, transpolyisoprene-based, fluoro rubber-based, chlorinated polyethylene-based thermoplastic elastomers are included, and a resin which is one kind thereof or is a combination of two or more kinds thereof (a polymer alloy, a polymer blend, a laminated body, or the like) can be used.

In addition, in the catheter main body 21, the vessel wall thereof may be a single layer or may be a laminated body in which a plurality of layers are laminated.

The connector unit 22 which is fixed to the proximal end portion of the catheter main body 21 is configured by a hard tubular body. This connector unit 22 is connected to the scanner apparatus 61 of the external unit 6.

Note that a method of fixing with respect to the catheter main body 21 of the connector unit 22 is not particularly limited, and for example a method by adhesion (adhesion by an adhesive agent and a solvent), a method by fusion (heat fusion, high frequency fusion, ultrasonic fusion, or the like), and the like are included.

In the middle in the longitudinal direction of the connector unit 22, a liquid injection port 221 which is diverged from the part is formed by projecting. For example, it is possible to inject the liquid Q from the liquid injection port 221 using a syringe. Then the injected liquid Q is filled in the sensor lumen 211 of the catheter main body 21.

A rotary support unit 222 which rotatably supports the driveshaft 3 is provided at the proximal end portion of the connector unit 22.

In addition, at the proximal end portion of the connector unit 22, a seal member 25 is arranged at the distal end side of the rotary support unit 222. The seal member 25 is configured by an elastic body in which the shape formed is ring-shaped. In this manner, the seal member 25 can prevent to generate a gap between the inner circumferential portion of the connector unit 22 and the outer circumferential portion of the driveshaft 3, that is, maintains liquidtighness, therefore, it is possible to prevent the liquid Q from leaking toward the proximal end direction.

The constituent materials of the connector unit 22 are not particularly limited, and for example, various kinds of resins such as polyvinyl chloride, polyethylene, polypropylene, cyclic polyolefin, polystyrene, poly-(4-methylpentene-1), polycarbonate, an acrylic resin, an acrylonitrile-butadiene-styrene copolymer, polyester such as polyethylene terephthalate and polyethylene naphthalate, a butadiene-styrene copolymer and polyamide (for example, nylon 6, nylon 6•6, nylon 6•10, and nylon 12) are included.

The connector unit 22 is a connector unit in which three tubular bodies are connected along the longitudinal direction in a configuration shown in Fig. 1, however, is not limited thereto, and for example, may be a connector unit which is configured by one tubular body.

The driveshaft 3 is inserted into the sensor lumen 211 of the catheter main body 21 of such the catheter 2. Then, the driveshaft 3 in this insertion state is rotated around the central axis thereof by a motion of the scanner apparatus 61.

The driveshaft 3 has a shaft 4 which is a body thereof and has a long shape, a housing 51 which is fixed to the distal end portion of the shaft 4, the ultrasonic vibrator 52 which is stored in the housing 51, a connector unit 53 which is fixed to the proximal end portion of the shaft 4. In addition, in the driveshaft 3, the housing 51, the ultrasonic vibrator 52, and the connector unit 53 configure an imaging means 5 for imaging an image of the blood vessel wall.

The shaft 4 has torque transmissibility which is capable of certainly transmitting a turning force by a motion of the scanner apparatus 61 to the ultrasonic vibrator 52. This shaft 4, for example, is formed by closely winding a metal wire such as stainless steel in a coil-shape (refer to Fig. 2). Note that this metal wire may be multiply-wound.

The outer diameter of this shaft 4 is smaller than the diameter of the sensor lumen 211. In this manner, a gap 213 is formed between the outer circumferential surface of the shaft 4 and the inner circumferential surface of the catheter main body 21. The liquid Q which is injected from a liquid injection port 221 of the catheter 2 flows down through the gap 213 and is discharged from the opening unit 212.

The housing 51 is fixed to the distal end portion of the shaft 4, for example, by an adhesive agent. The housing 51 is configured by a cylindrical body made from metal such as a stainless steel and a through hole 511 which penetrates through the wall part thereof is formed. From the through hole 511, the ultrasonic vibrator 52 is exposed. Note that the diameter of the housing 51 is substantially the same as the outer diameter of the shaft 4 or slightly larger than that.

The ultrasonic vibrator 52 is fixed in the housing 51 so that the center of gravity thereof is positioned on the central axis of the shaft 4. In this manner, the ultrasonic vibrator 52 can be rotated with the shaft 4. The ultrasonic vibrator 52 is formed in a rectangular or circular-shaped from, in a plane view and is an ultrasonic vibrator in which an electrode is formed on both surfaces of a piezoelectric body which is configured by PZT (lead zirconate titanate) by deposition, printing, or the like. In this manner, in the state facing onto the blood vessel wall, it is possible to emit ultrasonic waves from the ultrasonic vibrator 52 as well as it is possible to receive the reflected waves of the ultrasonic waves on the blood vessel wall, that is, it is possible to perform the transmission and reception of the ultrasonic waves . By this transmission and reception, it is possible to fetch an image of the blood vessel wall. This image is an image in which the ultrasonic waves is transmitted from the ultrasonic vibrator 52 and the distance to the blood vessel wall is measured in time until the reflected waves thereof return back to the ultrasonic vibrator 52 again to visualize the state of the blood vessel wall.

In addition, a plurality of signal lines (not shown) from the ultrasonic vibrator 52 are inserted into the shaft 4 to be electrically connected to the connector unit 53.

The connector unit 53 is connected to the scanner apparatus 61, and it is possible to directly receive a turning force from the scanner apparatus 61. This connector unit 53 is configured by a cylinder made of metal having conductivity such as copper. In this manner, it is also possible to electrically connect the connector unit 53 to the scanner apparatus 61, and it is possible to send an image signal from the ultrasonic vibrator 52 to the display unit 64 through the scanner apparatus 61. Then, an image of the blood vessel wall is displayed at the display unit 64.

Next, description will be given of the first tube 7. The first tube 7 is a medical tube in which the first site 71, the second site 72 and the third site 73 are provided in this order from the proximal end side toward the distal end side and which is made of a resin, as shown in Fig. 3

At the second site 72, the outer diameter c thereof is smaller than the outer diameter a of the first site 71 and the inner diameter d is substantially equal to the inner diameter b of the first site 71. Therefore, the thickness h of the second site 72 and the thickness i of the third site 73 are substantially equal.

At the third site 73, the outer diameter e and the inner diameter f thereof are respectively smaller than the outer diameter c and the inner diameter d of the second site 72.

In addition, the outer diameter a and the inner diameter b of the first site 71, the outer diameter c and the inner diameter d of the second site 72, and the outer diameter e and the inner diameter f of the third site 73 are respectively constant along the longitudinal direction of the first tube 7.

In addition, in a configuration shown in Figure, the thickness h of the second site 72 and the thickness i of the third site 73 are set to be substantially equal. Note that it goes without saying that the thickness h of the second site 72 and the thickness i of the third site 73 may be different.

In addition, the first tube 7 has a first taper unit 74 in which the outer diameter is gradually decreased from the proximal end side toward the distal end side between the first site 71 and the second site 72.

In addition, the first tube 7 has a second taper unit 75 in which the outer diameter and the inner diameter are gradually decreased from the proximal end side toward the distal end side between the second site 72 and the third site 73.

According to such the first tube 7, it is possible to make the outer diameter e of the third site 73 relatively small while making the outer diameter a of the first site 71 relatively large and making the inner diameter f of the third site 73 which has the smallest inner diameter sufficiently large. In this manner, the inner circumferential surface of the proximal end portion of the third tube 9 in which the inner diameter is relatively small can be certainly fixed to the outer circumferential surface of the distal end portion of the third site 73 of the first tube 7.

Note that the outer circumferences of the second site 72 and the third site 73 are the sites on which other tubes, that is, the second tube 8 and the third tube 9 are coated. The outer diameter of the second tube 8 which is provided on the outer circumference of the second site 72 and the outer diameter a of the first site 71 are substantially equal (refer to Fig. 2).

Here, the size of each unit of the first tube 7 is not, respectively, particularly limited, and is arbitrarily set according to the conditions, however, for example, the sizes as described below are preferable.

The outer diameter a of the first site 71 of the first tube 7 is preferably from approximately 1.02 mm to 1.10 mm, and more preferably from approximately 1.04 mm to 1.08 mm.

In addition, the inner diameter b of the first site 71 is preferably from approximately 0.70 mm to 0.78 mm, and more preferably from approximately 0.72 mm to 0.76 mm.

In addition, the outer diameter c of the second site 72 is preferably from approximately 0.82 mm to 0.90 mm, and more preferably from approximately 0.84 mm to 0.88 mm.

In addition, the inner diameter d of the second site 72 is preferably from approximately 0.70 mm to 0.78 mm, and more preferably from approximately 0.72 mm to 0.76 mm.

In addition, the outer diameter e of the third site 73 is preferably from approximately 0.71 mm to 0.79 mm, and more preferably from approximately 0.73 mm to 0.77 mm.

In addition, the inner diameter f of the third site 73 is preferably from approximately 0.62 mm to 0.68 mm, and more preferably from approximately 0.64 mm to 0.66 mm.

In addition, the thickness g of the first site 71 is preferably from approximately 0.12 mm to 0.19 mm, and more preferably from approximately 0.14 mm to 0.17 mm.

In addition, the thickness h of the second site 72 is preferably from approximately 0.02 mm to 0.10 mm, and more preferably from approximately 0.04 mm to 0.08 mm.

In addition, the thickness i of the third site 73 is preferably from approximately 0.01 m to 0.09 m, and more preferably from approximately 0.03 mm to 0.07 mm.

In addition, the thickness h of the second site 72 is preferably from approximately 11% to 83%, and more preferably from approximately 24% to 57%, of the thickness g of the first site 71.

In addition, the outer diameter c of the second site 72 is preferably from approximately 76% to 88%, and more preferably from approximately 79% to 85%, of the outer diameter a of the first site 71.

In addition, the outer diameter e of the third site 73 is preferably from approximately 79% to 96%, and more preferably from approximately 83% to 92%, of the outer diameter c of the second site 72.

In addition, the inner diameter f of the third site 73 is preferably from approximately 78% to 99%, and more preferably from approximately 83% to 93%, of the inner diameter d of the second site 72.

In addition, in a configuration shown in Figure 3, the outer diameter e of the third site 73 and the inner diameter d of the second site 72 are set to be substantially equal. That is, the third site 73 is shifted by the thickness i thereof to the inner circumferential side. Note that it goes without saying that the outer diameter e of the third site 73 and the inner diameter d of the second site 72 may be different.

Such the first tube 7 is a tube in which the first site 71, the second site 72, and the third site 73 are integrally formed of the same material.

In addition, the constituent materials of the first tube 7 are not particularly limited as long as the constituent materials are the resin materials having flexibility, and for example, thermoplastic resins such as polyolefin such as polyethylene and polypropylene, polyvinyl chloride, polyvinylidene chloride, polyurethane, polyvinylidene fluoride, polyolefin halide, polyethylene terephthalate, polybutylene terephthalate, polycarbonate, polyether ether ketone, a silicone rubber, and various kinds of thermoplastic elastomers such as styrene-based, polyolefin-based, polyurethane-based, polyester-based, polyamide-based, polyimide-based, polybutadiene-based, transpolyisoprene-based, fluoro rubber-based, and chlorinated polyethylene-based thermoplastic elastomers are included, and among these, a resin which is one kind thereof or a combination of two or more kinds thereof (a polymer alloy, a polymer blend, a laminated body, or the like) can be used. Note that, among these, polyether ether ketone is preferable. The reason is that polyether ether ketone is relatively hard and it is difficult for the coagulated blood to adhere when the blood coagulates.

Next, description will be given of a method of manufacturing the first tube 7.
[1] First, a tube for processing 70 which is a tube before processing is prepared, as shown in Fig. 4 (a). This tube for processing 70 has a definite outer diameter and a definite inner diameter. In addition, the outer diameter of the tube for processing 70 is substantially equal to the outer diameter a of the first site 71 of the first tube 7, the inner diameter is substantially equal to the inner diameter b of the first site 71, and the thickness is substantially equal to the thickness g of the first site 71.

### [2] First process

The outer circumferential surface of the distal end portion of the tube for processing 70 is physically or chemically ground, as shown in Fig. 4 (a). As a physical grinding, for example, a grinding apparatus, or the like is used. In addition, as a chemical grinding, for example, an etching, and the like are included.

In the first process, the site in which the tube for processing 70 is not ground becomes the first site 71 and the site which is ground becomes the second site 72. In addition, the first taper unit 74 is formed between the first site 71 and the second site 72.

### [3] Second process

Thermoforming is conducted, with respect to the site of the distal end side from the middle of the ground site of the tube for processing 70, and the outer diameter and the inner diameter of the site of the distal end side from the middle of the ground site are more reduced than those of the site of the proximal end side from the middle of the ground site, as shown in Fig. 3. The third site 73 and the second taper unit 75 are formed by thermoforming to obtain the first tube 7.

In addition, in thermoforming described above, a forming die having the inner diameter which is substantially equal to the outer diameter e of the third site 73 and a core bar having the outer diameter which is substantially equal to the inner diameter f of the third site 73 are used and heating is performed at the predetermined temperature. The heating temperature is not particularly limited, and is arbitrarily determined according to the conditions, however, for example, approximately 230°C to 270°C is preferable, and approximately 240°C to 260°C is more preferable.

In addition, the proximal end portion of the second tube 8 is adhered to the second site 72 of the first tube 7 and the first taper unit 74 by an adhesive agent, as shown in Fig. 5.

Specifically, a first adhesive agent 31 is provided at a joined part between the second tube 8 and the first tube 7, that is, between the proximal end surface of the second tube 8 and the first taper unit 74, and a second adhesive agent 32 is provided between the second tube 8 and the second site 72 in the vicinity of the joined part described above.

The first adhesive agent 31 and the second adhesive agent 32 are not particularly limited, however, it is preferred that a reaction system adhesive agent be used. As a reaction system adhesive agent, for example, a radiation curable adhesive agent which is cured by irradiating with radiation such as ultraviolet rays, and the like can be used.

When the proximal end portion of the second tube 8 is adhered to the second site 72 and the first taper unit 74, firstly, the second adhesive agent 32 is injected from a gap between the proximal end surface of the second tube 8 and the first taper unit 74 to be cured.

This second adhesive agent 32 is preferably an adhesive agent in which the viscosity is relatively low. In this manner, it is possible to inject the second adhesive agent 32 into more distal end side and enhance the joint strength between the second tube 8 and the second site 72.

As the second adhesive agent 32, for example, it is preferred that an acrylic resin-based adhesive agent, or the like be used.

Next, the first adhesive agent 31 is injected into a gap between the proximal end surface of the second tube 8 and the first taper unit 74 and is cured to seal the gap.

This first adhesive agent 31 is preferably an adhesive agent in which the viscosity is relatively high and which is chemical-resistant. In this manner, when the first adhesive agent 31 is injected into the gap described above, it is difficult for the first adhesive agent 31 to flow from the gap to other site, therefore, it is possible to certainly seal the gap. In addition, since the surface of the catheter main body 21 is treated by the predetermined chemicals, when the treatment is conducted, it is possible to certainly prevent the chemicals from entering into the gap described above.

As the first adhesive agent 31, for example, it is preferred that an epoxy resin-based adhesive agent, an urethane resin-based adhesive agent, or the like is used.

As explained above, according to the first tube 7, it is possible to make the outer diameter e of the third site 73 relatively small while making the outer diameter a of the first site 71 relatively large and making the inner diameter f of the third site 73 which has the smallest inner diameter sufficiently large. In this manner, the inner circumferential surface of the proximal end portion of the third tube 9 in which the outer diameter is relatively small can be certainly fixed to the outer circumferential surface of the distal end portion of the third site 73 of the first tube 7.

In addition, according the method of manufacturing the first tube 7, it is possible to easily and certainly manufacture the first tube 7.

Hereinbefore, descriptions of the medical tube, the catheter and the method of manufacturing the medical tube of the present invention have been given based on embodiments shown in Figures, however, the present invention is not limited thereto, and the configuration of each unit can be substituted by an arbitrary configuration having a similar function. In addition, other arbitrary structural component and process may be added to the present invention.

Furthermore, in the present invention, the catheter main body is not limited to a catheter having a distal end opening unit which is open at the distal end thereof as an outlet of a liquid, and may be, for example, a catheter main body having a side hole which penetrates into the side wall of the distal end portion.

In addition, in the present invention, an image which is obtained by the catheter assembly is not limited to an ultrasonic image, and may be, for example, an image which is optically obtained, that is, an image which is obtained by emitting and receiving the light. For example, the catheter may be a catheter which is used for an image by the light signal, in particular, an optical coherent tomography apparatus (OCT), and an optical frequency domain imaging apparatus (OFDI) which is an improved version thereof. In this case, after a interference light is generated by irradiating a biological tissue with near infrared rays which are emitted from the distal end portion of the driveshaft and interfering a reflected light from the biological tissue with a reference light, it is possible to generate a cross-sectional image in the body lumen such as the blood vessel based on the interference light.

### Industrial Applicability

According to the present invention, a medical tube in which the outer diameter of the proximal end side is relatively large, and the outer diameter of the distal end side is relatively small while securing the inner diameter having sufficient size at the distal end side can be provided. In addition, according to the present invention, such a medical tube can be easily and certainly manufactured. Therefore, the invention is susceptible of industrial application.

### Explanation of Reference

- 1: CATHETER ASSEMBLY
- 2: CATHETER
- 21: CATHETER MAIN BODY
- 210: DRIVESHAFT INSERTION UNIT
- 211: SENSOR LUMEN
- 212: OPENING UNIT
- 213: GAP
- 22: CONNECTOR UNIT
- 221: LIQUID INJECTION PORT
- 222: ROTARY SUPPORT UNIT
- 23: GUIDE WIRE INSERTION UNIT
- 231,232: INSERTION MEMBER
- 233: GUIDE WIRE LUMEN
- 25: SEAL MEMBER
- 3: DRIVESHAFT
- 4: SHAFT
- 5: IMAGING MEANS
- 51: HOUSING
- 511: THROUGH HOLE
- 52: ULTRASONIC VIBRATOR (IMAGE FETCHING UNIT)
- 53: CONNECTOR UNIT
- 6: EXTERNAL UNIT
- 61: SCANNER APPARATUS
- 62: AXIAL DIRECTION MOVING APPARATUS
- 63: CONTROL UNIT
- 64: DISPLAY UNIT
- 7: FIRST TUBE
- 70: TUBE FOR PROCESSING
- 71: FIRST SITE
- 72: SECOND SITE
- 73: THIRD SITE
- 74: FIRST TAPER UNIT
- 75: SECOND TAPER UNIT
- 8: SECOND TUBE
- 9: THIRD TUBE
- 31: FIRST ADHESIVE AGENT
- 32: SECOND ADHESIVE AGENT
- 200: GUIDE WIRE
- Q: LIQUID

## Claims

1. A medical tube which is provided with a first site (71), a second site (72) and a third site (73) in this order from the proximal end side toward the distal end side and is made of resin,
wherein at the second site (72), the outer diameter thereof is smaller than the outer diameter of the first site (71) and the inner diameter is substantially equal to the inner diameter of the first site (71), and
wherein at the third site (73), the outer diameter and the inner diameter thereof are respectively smaller than the outer diameter and the inner diameter of the second site, **characterized in that** the first site (71), the second site (72), and the third site (73) are integrally formed of the same material.

2. The medical tube according to claim 1, comprising: a first taper unit (74) of which the outer diameter is gradually decreased from the proximal end side toward the distal end side between the first site (71) and the second site (72).

3. The medical tube according to claims 1 or 2, comprising: a second taper unit (75) of which the outer diameter and the inner diameter are, respectively, gradually decreased from the proximal end side toward the distal end side between the second site (72) and the third site (73).

4. The medical tube according to any one of claims 1 to 3,
wherein an outer circumference of the second site and third site is a site on which other tube is coated.

5. The medical tube according to any one of claims 1 to 3,
wherein the wall thickness (h) of the second site (72) and the wall thickness (i) of the third site (73) are substantially equal.

6. The medical tube according to any one of claims 1 to 5,
wherein the wall thickness of the second site (72) is 11% to 83% of the wall thickness of the first site (71).

7. The medical tube according to any one of claims 1 to 6,
wherein the outer diameter of the second site (72) is 76% to 88% of the outer diameter of the first site (71).

8. The medical tube according to any one of claims 1 to 7,
wherein the outer diameter of the third site (73) is 79% to 96% of the outer diameter of the second site (72).

9. The medical tube according to any one of claims 1 to 8,
wherein the inner diameter of the third site (73) is 78% to 99% of the inner diameter of the second site (72).

10. The medical tube according to any one of claims 1 to 9,
wherein the outer diameter of the third site (73) and the inner diameter of the second site (72) are substantially equal.

11. The medical tube according to any one of claims 1 to 10,
wherein the medical tube is configured by a thermoplastic resin and has flexibility.

12. A catheter (2) comprising:
a catheter main body (21) which is insertable into a body lumen to be used and has flexibility,
wherein the catheter main body (21) has the medical tube according to any one of claims 1 to 11.

13. A method of manufacturing the medical tube according to any one of claims 1 to 11, comprising:
a first process of physically or chemically grinding the outer circumferential surface of the distal end portion of a tube (70) for processing; and
a second process for conducting thermoforming, with respect to the site of the distal end side from a middle of a ground site of the tube for processing (70), and more reducing the outer diameter and the inner diameter of the site of the distal end side from the middle of the ground site than those of the site of the proximal end side from the middle of the ground site,
wherein in the first process, the site which is not ground becomes the first site (71) and the site which is ground becomes the second site (72), and
wherein in the second process, the third site (73) is formed by performing the thermoforming.

## Patentansprüche

1. Medizinischer Schlauch, der mit einer ersten Stelle (71), einer zweiten Stelle (72) und einer dritten Stelle (73) in dieser Reihenfolge von der proximalen Stirnseite zur distalen Stirnseite hin versehen ist und aus Harz besteht,
wobei an der zweiten Stelle (72) der Außendurchmesser derselben kleiner ist als der Außendurchmesser der ersten Stelle (71) und der Innendurchmesser im Wesentlichen gleich dem Innendurchmesser der ersten Stelle (71) ist, und
wobei an der dritten Stelle (73) der Außendurchmesser und der Innendurchmesser derselben jeweils kleiner sind als der Außendurchmesser und der Innendurchmesser der zweiten Stelle, **dadurch gekennzeichnet, dass** die erste Stelle (71), die zweite Stelle (72) und die dritte Stelle (73) einstückig aus demselben Material gebildet sind.

2. Medizinischer Schlauch nach Anspruch 1, umfassend: eine erste Konuseinheit (74), deren Außendurchmesser von der proximalen Stirnseite zur distalen Stirnseite hin zwischen der ersten Stelle (71) und der zweiten Stelle (72) allmählich abnimmt.

3. Medizinischer Schlauch nach Anspruch 1 oder 2, umfassend: eine zweite Konuseinheit (75), deren Außendurchmesser und Innendurchmesser jeweils von der proximalen Stirnseite zur distalen Stirnseite hin zwischen der zweiten Stelle (72) und der dritten Stelle (73) allmählich abnehmen.

4. Medizinischer Schlauch nach einem der Ansprüche 1 bis 3, wobei ein Außenumfang der zweiten Stelle und dritten Stelle eine Stelle ist, an der ein anderer Schlauch aufgebracht ist.

5. Medizinischer Schlauch nach einem der Ansprüche 1 bis 3, wobei die Wandstärke (h) der zweiten Stelle (72) und die Wandstärke (i) der dritten Stelle (73) im Wesentlichen gleich sind.

6. Medizinischer Schlauch nach einem der Ansprüche 1 bis 5, wobei die Wandstärke der zweiten Stelle (72) 11% bis 83% der Wandstärke der ersten Stelle (71) beträgt.

7. Medizinischer Schlauch nach einem der Ansprüche 1 bis 6, wobei der Außendurchmesser der zweiten Stelle (72) 76% bis 88% des Außendurchmessers der ersten Stelle (71) beträgt.

8. Medizinischer Schlauch nach einem der Ansprüche 1 bis 7, wobei der Außendurchmesser der dritten Stelle (73) 79% bis 96% des Außendurchmessers der zweiten Stelle (72) beträgt.

9. Medizinischer Schlauch nach einem der Ansprüche 1 bis 8, wobei der Innendurchmesser der dritten Stelle (73) 78% bis 99% des Innendurchmessers der zweiten Stelle (72) beträgt.

10. Medizinischer Schlauch nach einem der Ansprüche 1 bis 9, wobei der Außendurchmesser der dritten Stelle (73) und der Innendurchmesser der zweiten Stelle (72) im Wesentlichen gleich sind.

11. Medizinischer Schlauch nach einem der Ansprüche 1 bis 10, wobei der medizinische Schlauch aus einem thermoplastischen Harz gebildet ist und Flexibilität besitzt.

12. Katheter (2), umfassend:
einen Katheterhauptkörper (21), der in ein zu verwendendes Körperlumen eingeführt werden kann und Flexibilität besitzt, wobei der Katheterhauptkörper (21) den medizinischen Schlauch nach einem der Ansprüche 1 bis 11 aufweist.

13. Verfahren zur Herstellung des medizinischen Schlauches nach einem der Ansprüche 1 bis 11, umfassend:
einen ersten Vorgang des physikalischen oder chemischen Schleifens der äußeren Umfangsfläche des distalen Endabschnitts eines zu verarbeitenden Schlauches (70); und
einen zweiten Vorgang zur Durchführung des Warmformens in Bezug auf die Stelle der distalen Stirnseite ab einer Mitte einer geschliffenen Stelle des zu verarbeitenden Schlauches (70) und zur Reduzierung des Außendurchmessers und des Innendurchmessers der Stelle der distalen Stirnseite ab der Mitte der geschliffenen Stelle mehr als den Außen- und Innendurchmesser der Stelle der proximalen Stirnseite ab der Mitte der geschliffenen Stelle,
wobei bei dem ersten Vorgang die Stelle, die nicht geschliffen ist, zu der ersten Stelle (71) wird und die Stelle, die geschliffen ist, zu der zweiten Stelle (72) wird, und
wobei bei dem zweiten Vorgang die dritte Stelle (73) gebildet wird, indem das Warmformen durchgeführt wird.

## Revendications

1. Tube médical qui est muni d'un premier site (71), d'un second site (72) et d'un troisième site (73) dans cet ordre en allant du côté de l'extrémité proximale vers le côté de l'extrémité distale et est fait de résine,
où au niveau du second site (72), son diamètre extérieur est inférieur au diamètre extérieur du premier site (71) et le diamètre intérieur est sensiblement égal au diamètre intérieur du premier site (71), et
où au niveau du troisième site (73), son diamètre extérieur et son diamètre intérieur sont respectivement inférieurs au diamètre extérieur et au diamètre intérieur du second site, **caractérisé en ce que** le premier site (71), le second site (72) et le troisième site (73) sont formés intégralement du même matériau.

2. Tube médical selon la revendication 1, comprenant : une première unité conique (74) dont le diamètre extérieur est progressivement réduit depuis le côté de l'extrémité proximale vers le côté de l'extrémité distale entre le premier site (71) et le second site (72).

3. Tube médical selon la revendication 1 ou 2, comprenant : une seconde unité conique (75) dont le diamètre extérieur et le diamètre intérieur sont, respectivement, progressivement réduits depuis le côté de l'extrémité proximale vers le côté de l'extrémité distale entre le second site (72) et le troisième site (73).

4. Tube médical selon l'une quelconque des revendications 1 à 3,
dans lequel une circonférence extérieure du second site et du troisième site est un site sur lequel un autre tube est appliqué.

5. Tube médical selon l'une quelconque des revendications 1 à 3,
dans lequel l'épaisseur de paroi (h) du second site (72) et l'épaisseur de paroi (i) du troisième site (73) sont sensiblement égales.

6. Tube médical selon l'une quelconque des revendications 1 à 5,
dans lequel l'épaisseur de paroi du second site (72) est de 11% à 83% de l'épaisseur de paroi du premier site (71).

7. Tube médical selon l'une quelconque des revendications 1 à 6,
dans lequel le diamètre extérieur du second site (72) est de 76% à 88% du diamètre extérieur du premier site (71).

8. Tube médical selon l'une quelconque des revendications 1 à 7,
dans lequel le diamètre extérieur du troisième site (73) est de 79% à 96% du diamètre extérieur du second site (72).

9. Tube médical selon l'une quelconque des revendications 1 à 8,
dans lequel le diamètre intérieur du troisième site (73) est de 78% à 99% du diamètre intérieur du second site (72).

10. Tube médical selon l'une quelconque des revendications 1 à 9,
dans lequel le diamètre extérieur du troisième site (73) et le diamètre intérieur du second site (72) sont sensiblement égaux.

11. Tube médical selon l'une quelconque des revendications 1 à 10,
dans lequel le tube médical est fait d'une résine thermoplastique et est flexible.

12. Cathéter (2) comprenant :
un corps principal de cathéter (21) qui peut être inséré dans une lumière corporelle à utiliser et qui est flexible,
dans lequel le corps principal de cathéter (21) comprend le tube médical selon l'une quelconque des revendications 1 à 11.

13. Procédé de fabrication du tube médical selon l'une quelconque des revendications 1 à 11,
comprenant :
une première opération consistant à rectifier physiquement ou chimiquement la surface circonférentielle extérieure de la partie d'extrémité distale d'un tube (70) à traiter ; et
une seconde opération consistant à procéder au thermoformage, par rapport au site du côté de l'extrémité distale à partir du milieu d'un site rectifié du tube à traiter (70), et réduire le diamètre extérieur et le diamètre intérieur du site du côté de l'extrémité distale à partir du milieu du site rectifié davantage que ceux du site du côté de l'extrémité proximale à partir du milieu du site rectifié,
dans lequel dans la première opération, le site qui n'est pas rectifié devient le premier site (71) et le site qui est rectifié devient le second site (72), et
dans lequel dans la seconde opération, le troisième site (73) est formé en procédant au thermoformage.
